(19) Europäisches Patentamt
European Patent Office
Office européen des brevets

(11) Publication number: **0 073 849**
**A1**

(12) **EUROPEAN PATENT APPLICATION**

(21) Application number: 81106891.5

(22) Date of filing: 03.09.81

(51) Int. Cl.[3]: C 07 D 209/86
C 07 D 209/88, A 61 K 31/40

(43) Date of publication of application:
16.03.83 Bulletin 83/11

(84) Designated Contracting States:
AT BE CH DE FR GB IT LI NL SE

(71) Applicant: SCHERING CORPORATION
2000 Galloping Hill Road
Kenilworth, New Jersey 07033(US)

(72) Inventor: Tahbaz, Pirouz
96 Oak Drive
Cedar Grove New Jersey 07009(US)

(74) Representative: Antony, Fritz, Dr. et al,
P.O. Box 601 Winkelriedstrasse 35
CH-6002 Lucerne(CH)

(54) **Novel phenyl-1,2,3,4-tetrahydrocarbazoles, their preparation and pharmaceutical compositions containing them.**

(57) Novel phenyl-1,2,3,4,-tetrahydrocarbazoles are anti-depressants useful in the treatment of mental depression of either endogenous or reactive nature. Processes for their preparation and pharmaceutical compositions containing them are also disclosed.

EP 0 073 849 A1

0073849

Case 2200
July 17, 1981
JB/vl

Novel phenyl-1,2,3,4-tetrahydrocarbazoles, their preparation and pharmaceutical compositions containing them.

This invention relates to phenyl-1,2,3,4-tetrahydrocarbazoles, to pharmaceutical compositions containing them, and to their preparation. The novel compounds are anti-depressants useful in the treatment of mental depression of either endogenous or reactive nature.

According to the invention we provide phenyl-1,2,3,4-tetrahydrocarbazoles of the formula

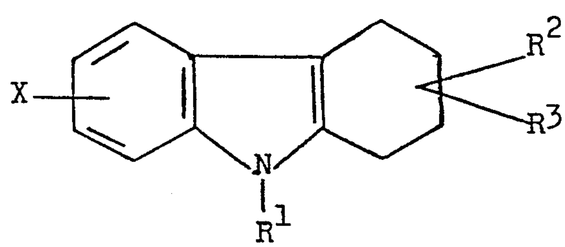

I

wherein X is a hydrogen or halogen atom or a nitro, $NR^4R^5$, lower alkyl, hydroxy, lower alkoxy or trifluoromethyl group;

$R^1$ is a hydrogen atom or a lower alkyl, $NR^4R^5$-substituted lower alkyl or $NR^4R^5$-CO group;

$R^3$ is a phenyl or X-substituted phenyl group, where X is as defined above;

$R^2$ is a hydrogen atom or an $NR^4R^5$-substituted alkyl, cyano,

$NR^4R^5$-CO or COOR group, where R is a hydrogen atom or a lower alkyl group;

with the proviso that $R^2$ and $R^3$ are located on the same carbon atom;

and $R^4$ and $R^5$, which may be the same or different, are hydrogen atoms or lower alkyl groups;

where the term "lower" indicates groups having 1 to 6 carbon atoms;

and the pharmaceutically acceptable salts of those compounds having an acidic or basic group.

Lower alkyl and lower alkoxy groups may be straight or branched, e.g., n-propyl and t-butoxy, but preferably methyl, ethyl or methoxy. Halogen atoms may be fluorine, chlorine, bromine or iodine, but chlorine, fluorine and bromine are preferred. The $NR^4R^5$-substituted alkyl groups may have a straight or branched alkyl chain but preferably have the formula $NR^4R^5-(CH_2)_n$ wherein n is an integer from 1 to 6. The radicals $R^2$ and $R^3$ may be located on any one of the carbon atoms in position 1-, 2-, 3- or 4- of the 1,2,3,4-tetrahydrocarbazole nucleus but are preferably located on the carbon atom in the 3-position.

Those compounds of the formula I that are acidic (by virtue of COOH in $R^2$) or basic (by virtue of $NR^4R^5$ in X or $NR^4R^5$-substituted alkyl in $R^1$ or $R^2$) can be isolated as the free acid or base or as salts thereof. Salts of the acidic compounds in particular include pharmaceutically acceptable

salts such as their sodium, potassium, ammonium, magnesium and calcium salts. Salts of the basic compounds include their pharmaceutically acceptable salts with mineral or organic acids such as hydrochloric, hydrobromic, sulphuric, phosphoric, fumaric, maleic, citric, tartaric, succinic or adipic acid.

Since the radicals $R^2$ and $R^3$ are different, the compounds of the formula I exist as racemates and as the resolved optically active forms, and the present invention relates to all of these. The compounds can be resolved by standard methods: e.g., the compounds that form salts can be resolved by salt-formation with an appropriate optically active acid or base and fractional crystallisation.

The compounds of the formula I can be prepared by standard methods. In particular, the 1,2,3,4-tetrahydrocarbazole nucleus can be prepared by condensation of a phenylhydrazine with a substituted cyclohexanone under standard conditions for a Fischer-Indole synthesis. The invention therefore provides a process for the preparation of a compound of the formula I defined above, which comprises either (a) condensing a phenylhydrazine of the formula

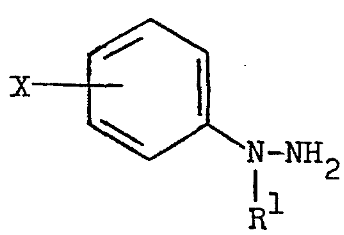

II

with a cyclohexanone of the formula

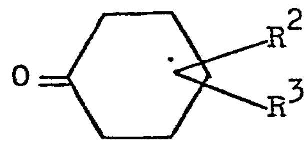

III

in an acidic medium at moderately elevated temperature, <u>or</u> (b) cyclising the intermediary phenylhydrazone of the formula

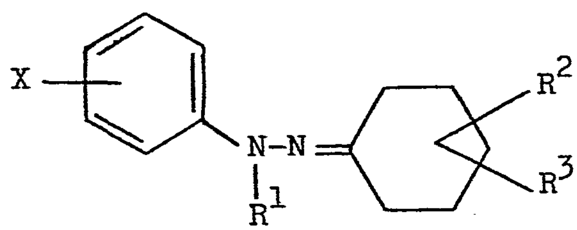

IV

in an acidic medium at elevated temperature. In these processes, the radicals X, $R^1$, $R^2$ and $R^3$ are as defined for formula I.

In process a), at least one equivalent excess of acid is preferably used, so that the intermediary phenylhydrazone of formula IV cyclises *in situ* to the 1,2,3,4-tetrahydrocarbazole of the formula I. If no excess of acid is used, the phenylhydrazone of formula IV can be isolated and then cyclised with excess acid to the 1,2,3,4-tetrahydrocarbazole of formula I. In either process a) or b), the reactants of the formula II and III or the phenylhydrazone of the formula IV are heated in an acidic medium for an approprionate time, e.g. half-an-hour to 24 hours. The acid may be inorganic or

organic, e.g. sulfuric, phosphoric, hydrochloric, hydrobromic, acetic or methanesulfonic acid, or a Lewis acid e.g. boron trifluoride; in particular the reactions can often conveniently be carried out by heating in ethanolic hydrochloric acid or in acetic acid at reflux for about an hour.

Products of the formula I that are acidic or basic can if desired be isolated as salts as hereinabove defined.

The starting materials of the formulae II and III are in general well-known. Any particular starting material that is not known or not immediately available can be prepared by standard methods.

Although the processes described above are generally known for the preparation of 1,2,3,4-tetrahydrocarbazoles, it is sometimes more convenient not to have a desired radical $R^1$ present in the phenylhydrazine starting material but rather introduce it after the cyclisation. Equally it may be more convenient to use a starting material of the formula II or III wherein X or $R^2$ differs from the X or $R^2$ radical desired in the product of the formula I, and modify the X or $R^2$ radical after a compound of the formula I has been prepared. Examples of such finishing steps are:

a) Reduction of a nitro group in X to an unsubstituted amino group. The reduction can be effected under standard conditions, preferably by means of hydrogen and a catalyst (e.g. palladium on carbon) in an inert organic solvent. In particular, the reduction can be effected in a lower alkanol, especially ethanol or methanol, as solvent at room temperature over a period of a few days.

b) Dealkylation of a lower alkoxy group, especially a methoxy group, in X to a hydroxy group. This reaction can be effected under standard conditions, for example with a Lewis acid, especially boron trifluoride, in an inert anhydrous organic solvent such as diethyl ether.

c) A 9-unsubstituted 1,2,3,4-tetrahydrocarbazole of the formula I can be N-alkylated under standard conditions to introduce an alkyl or $NR^4R^5$-substituted alkyl group, for example by reaction with an alkyl or $NR^4R^5$-substituted alkyl halide (e.g. bromide or chloride) or other reactive ester (e.g. methanesulfonate). This reaction is preferably carried out in an inert, non-hydroxylic polar solvent such as dimethylformamide in the presence of a strong non-hydroxylic base such as sodium hydride or sodamide at elevated temperature (e.g. 75-85°C.) for a half to 8 hours, preferably about 2 hours.

d) Similarly a 9-$CONH_2$ or 9-$CONHR^4$ group (wherein $R^4$ is a lower alkyl group) can be introduced into a 9-unsubstituted 1,2,3,4-tetrahydrocarbazole of formula I by reaction of the last-named with chlorosulfonyl isocyanate or with a lower alkyl isocyanate, preferably at about room temperature, in an inert organic solvent such as an ether, and alkaline hydrolysis of the resulting 9-chlorosulfonylcarbamoyl compound when chlorosulfonylisocyanate has been used. This hydrolysis can be effected by a strong alkali such as potassium or ammonium hydroxide in an aqueous organic solvent such as aqueous acetone.

e) A group CN as $R^2$ can be hydrated to $CONH_2$ or hydrolysed

to COOH. The hydration can be effected in concentrated sulfuric acid at elevated temperature (e.g. about 100°C.). The hydrolysis can be effected by heating in a high-boiling solvent, such as ethylene glycol, in the presence of an alkali metal hydroxide.

f) A group CN as $R^2$ can also be converted into an alkoxycarbonyl group by hydrolysis according to step e) and esterification of the resulting carboxylic acid, e.g. with the desired alkanol ROH and an esterifying or dehydrating agent such as an acid at elevated temperature.

g) A group CN as $R^2$ can also be converted into a group $CH_2NH_2$ by reduction, preferably with a complex metal hydride in an ether solvent, especially with lithium aluminium hydride in tetrahydrofuran.

When the substituents $R^2$ and $R^3$ are on the 2- or 4-carbon atom of the cyclohexanone of the formula III and when X is at the 2- or 4-position of the phenylhydrazine, only a single racemic product will be obtained. However, if the substituents $R^2$ and $R^3$ are on the 3-carbon atom of the cyclohexanone of the formula III, or if the substituent X is at the 3-position of the phenylhydrazine, then the product of the formula I will normally be a mixture of racemic 2-$R^2$-2-$R^3$- and 4-$R^2$-4-$R^3$-1,2,3,4-tetrahydrocarbazoles or of 5X- and 7X--1,2,3,4-tetrahydrocarbazoles, which can be separated by standard methods such as chromatography and/or fractional crystallisation.

The following examples illustrate the invention:

## EXAMPLE I

### 6-Methoxy-3-phenyl-1,2,3,4-tetrahydrocarbazole

To a mixture of 4-methoxy-phenylhydrazine hydrochloride (50.0 g.) and 4-phenylcyclohexanone (50.0 g.) add 500 ml. of acetic acid and with constant stirring heat the mixture for one hour at 80-90°C. Cool the mixture, add 200 ml. of water and filter off and dry the resulting precipitate. Recrystallize the product from methanol/water (1:1), m.p. 114-115°.

Replacement of the 4-methoxyphenylhydrazine hydrochloride in the procedure of the foregoing Example with equivalent quantities of the hydrochloride salt of the following X-substituted phenylhydrazines:

2-ethylphenylhydrazine,

4-methylphenylhydrazine,

4-nitrophenylhydrazine,

4-chlorophenylhydrazine,

4-ethoxyphenyl hydrazine,

phenylhydrazine,

3,4-dimethoxyphenyl hydrazine and

3-methoxyphenylhydrazine

yields

8-ethyl-3-phenyl-1,2,3,4-tetrahydrocarbazole,

6-methyl-3-phenyl-1,2,3,4-tetrahydrocarbazole (m.p. 98-101°C.),

6-nitro-3-phenyl-1,2,3,4-tetrahydrocarbazole (m.p. 195-196°C.),

6-chloro-3-phenyl-1,2,3,4-tetrahydrocarbazole,

6-ethoxy-3-phenyl-1,2,3,4-tetrahydrocarbazole,

3-phenyl-1,2,3,4-tetrahydrocarbazole (m.p. 125-126°C.),
6,7-dimethoxy-3-phenyl-1,2,3,4-tetrahydrocarbazole, and
5-methoxy-3-phenyl-1,2,3,4-tetrahydrocarbazole and/or
7-methoxy-3-phenyl-1,2,3,4-tetrahydrocarbazole.

Similarly replacement of the 4-phenylcyclohexanone in the procedure of the foregoing example with equivalent quantities of the following X-phenyl-$R^2$-cyclohexanones:
4-cyano-4-methoxyphenyl-cyclohexanone,
4-cyano-4-phenyl-cyclohexanone,
4-carboxy-4-phenyl-cyclohexanone,
4-cyano-4-(4-methylphenyl)-cyclohexanone,
4-chlorophenyl-cyclohexanone, and
4-fluorophenyl-cyclohexanone,
yields
3-cyano-3-(4-methoxyphenyl)-6-methoxy-1,2,3,4-tetrahydrocarbazole,
3-cyano-3-phenyl-6-methoxy-1,2,3,4-tetrahydrocarbazole (m.p. 189-190°C.),
3-carboxy-3-phenyl-6-methoxy-1,2,3,4-tetrahydrocarbazole (m.p. 198-199°C.),
3-cyano-3-(4-methylphenyl)-6-methoxy-1,2,3,4-tetrahydrocarbazole,
3-(4-chlorophenyl)-6-methoxy-1,2,3,4-tetrahydrocarbazole, and
3 (4-fluorophenyl)-6-methoxy-1,2,3,4-tetrahydrocarbazole.

Similarly replacement of the 4-phenylcyclohexanone with equivalent quantities of the following X-phenyl-$R^2$-cyclohexanones:
4-cyano-4-methoxyphenyl-cyclohexanone,

4-cyano-4-phenyl-cyclohexanone,
4-carboxy-4-phenyl-cyclohexanone,
4-cyano-4-(4-methylphenyl)-cyclohexanone,
4-chlorophenyl-cyclohexanone, and
4-fluorophenyl-cyclohexanone,
together with replacement of the 4-methoxy-phenylhydrazine hydrochloride with phenylhydrazine hydrochloride yields
3-cyano-3-(4-methoxyphenyl)-1,2,3,4-tetrahydrocarbazole,
3-cyano-3-phenyl-1,2,3,4-tetrahydrocarbazole,
3-carboxy-3-phenyl-1,2,3,4-tetrahydrocarbazole,
3-cyano-3-(4-methylphenyl)-1,2,3,4-tetrahydrocarbazole,
3-(4-chlorophenyl)-1,2,3,4-tetrahydrocarbazole, and
3-(4-fluorophenyl)-1,2,3,4-tetrahydrocarbazole.

The following compounds of formula I can be prepared similarly:

6-chloro-3-(3-fluorophenyl)-1,2,3,4-tetrahydrocarbazole (m.p. 121-123°C.);
6-chloro-4-cyano-3-phenyl-1,2,3,4-tetrahydrocarbazole (m.p. 196-196.5°C.),
6-chloro-3-(4-chlorophenyl)-1,2,3,4-tetrahydrocarbazole (m.p. 139-140°C.),
6-methoxy-3-(4-methoxyphenyl)-1,2,3,4-tetrahydrocarbazole (m.p. 151-153°C.),
6-methoxy-3-phenyl-9-methyl-1,2,3,4-tetrahydrocarbazole (m.p. 93-94°C.),
6-methoxy-3-(4-chlorophenyl)-1,2,3,4-tetrahydrocarbazole (m.p. 92-93°C.),

6-methoxy-3-(4-methylphenyl)-1,2,3,4-tetrahydrocarbazole (m.p. 140-141°C.), and

6-chloro-3-(4-methoxyphenyl)-1,2,3,4-tetrahydrocarbazole.

6-Hydroxy-3-phenyl-1,2,3,4-tetrahydrocarbazole (m.p. 76-79°C.) can be prepared from the main compound of this Example by O-dealkylation e.g. with boron trifluoride in diethyl ether.

EXAMPLE II

6-Methoxy-3-aminomethyl-3-phenyl-1,2,3,4-tetrahydrocarbazole hydrochloride.

To 6-methoxy-3-cyano-3-phenyl-1,2,3,4-tetrahydrocarbazole (1.0 g.) in tetrahydrofuran (10 ml.) is added lithium aluminium hydride (0.4 g.) and the mixture is refluxed for one hour. Then 0.1 ml. of water is cautiously added and the resulting mixture is filtered. The filtrate is evaporated to dryness, the residue dissolved in methylene chloride and then ethereal hydrogen chloride is added to precipitate the desired compound, m.p. 175-178°C.

EXAMPLE III

3-phenyl-6-methoxy-9-carbamoyl-1,2,3,4-tetrahydrocarbazole

Chlorosulfonyl isocyanate (2.8 g.) in ether (10 ml.) is slowly added to a cold solution (0-10°C.) of 3-phenyl-6-methoxy-1,2,3,4-tetrahydrocarbazole (5.5 g.) in ether (50 ml.) and the resulting solution is stirred at room temperature for two and a half hours, and then evaporated to dryness. A 1:7 mixture of water and acetone (80 ml.) is added to the

residue followed by 10% $NH_4OH$ (50 ml.) and the mixture is stirred at room temperature for half an hour. The solid obtained thereby is recrystallized from ethyl acetate to yield the desired product, m.p. 188-189°C.

EXAMPLE IV

6-Methoxy-3-carboxy-3-phenyl-1,2,3,4-tetrahydrocarbazole

A mixture of 6-methoxy-3-cyano-3-phenyl-1,2,3,4-tetrahydrocarbazole (48.0 g.) and potassium hydroxide (60.0 g.) in ethylene glycol (300 ml.) is heated at 170°C. for 16 hours and then cooled. To the cooled solution dilute hydrochloric acid is added to bring the mixture to pH 8. The resulting precipitate is recrystallized from acetonitrile to give the desired compound, m.p. 198-199°C.

6-Chloro-3-carboxy-3-phenyl-1,2,3,4-tetrahydrocarbazole (m.p. 227-228°C.) can be prepared similarly from 6-chloro-3-cyano-3-phenyl-1,2,3,4-tetrahydrocarbazole.

6-Methoxy-3-ethoxycarbonyl-3-phenyl-1,2,3,4-tetrahydrocarbazole (m.p. 137-138°C.) can be prepared by esterification of the compound of this Example under standard conditions.

EXAMPLE V

6-Methoxy-3-phenyl-9-(3-dimethylaminopropyl)-1,2,3,4-tetrahydrocarbazole hydrochloride

A mixture of 6-methoxy-3-phenyl-1,2,3,4-tetrahydrocarbazole (5.5 g.) and 50% sodium hydride (1.0 g.) in dimethylformamide

(30 ml.) is heated at 100°C. for one hour and a half and then 3-dimethylaminopropyl chloride (2.5 g.) is added. The mixture is heated at 80°C. for two hours and then cooled, and water (40 ml.) is added. The solid which precipitates is recrystallized from methylene chloride:ether (1:1); its hydrochloride melts at 90-98°C.

6-Methoxy-3-phenyl-3-ethoxycarbonyl-9-(3-dimethylaminopropyl)-1,2,3,4-tetrahydrocarbazole hydrochloride (m.p. 90-95°C.) and 6-chloro-3-(4-chlorophenyl)-9-(3-dimethylamino--propyl)-1,2,3,4-tetrahydrocarbazole hydrochloride (m.p. 247-249°C.) can be prepared similarly.

6-Methoxy-3-phenyl-9-methyl-1,2,3,4-tetrahydrocarbazole (m.p. 93-94°C.) can be prepared by similar N-methylation.

EXAMPLE VI

6-Chloro-3-carbamoyl-3-phenyl-1,2,3,4-tetrahydrocarbazole

6-Chloro-3-cyano-3-phenyl-1,2,3,4-tetrahydrocarbazole (10 g.) was heated in concentrated sulfuric acid (30 ml.) at 90-100°C. for one hour and a half. The solution was cooled and poured into ice water and the precipitate was filtered off, washed with water and dried. The product was recrystallised from methylene chloride; m.p. 140-142°C.

EXAMPLE VII

6-Amino-3-(4-methoxyphenyl)-1,2,3,4-tetrahydrocarbazole

6-Nitro-3-(4-methoxyphenyl)-1,2,3,4-tetrahydrocarbazole (2 g.) in ethanol (100 ml.) was hydrogenated by means of

palladium on carbon (300 mg.) for 3 days at room temperature. The catalyst was then filtered off and washed with a little ethanol, the filtrate and washings were evaporated, and the residue recrystallized (aq. ethanol); m.p. 202-204$^{\circ}$C. (dec.).

As stated above, the compounds of formula I are anti-depressants useful in the treatment of mental depression of either endogenous or reactive nature.

The anti-depressant activity of the compounds of formula I may be ascertained by standard biological tests (e.g., the rat muricide test). Compounds of formula I are tested not only for their absolute activity but also by comparison with such well-known standards as amitriptyline and imipramine. These tests show that the compounds of the formula I exert their anti-depressant activity in the treatment of mental depression in humans at dosage levels of about 0.5 to 6 mg./kg. per day, preferably in 3-4 divided doses. Preferably the compounds of this invention are administered in amounts of 25-300 mg. per day in 3-4 divided doses, i.e. in dosage units of 6 to 100 mg.

Other tests indicate that the compounds of formula I in general should have a relatively fast onset of action and should exert low or very low anticholinergic side effects, when compared to such standards as imipramine or amitriptyline.

The invention therefore provides pharmaceutical compositions containing, as active ingredient, at least one compound of the formula I, or a pharmaceutically acceptable salt of

such a compound having an acidic or basic group, together with a pharmaceutical carrier or excipient. Such compositions can be prepared by bringing the active ingredient into a form suitable for therapeutic administration, in particular by admixing with a pharmaceutical carrier or excipient.
For use as anti-depressants the compounds of the formula I may thus be formulated by techniques well known in the art as standard pharmaceutical compositions suitable for oral, rectal or parenteral administration, for example tablets, capsules, aqueous or oily suspensions or solutions, emulsions, powders or suppositories. Suitable adjuvants such as calcium carbonate, starch, lactose, talc, magnesium stearate and gum acacia can advantageously be used.

Preferred compounds of formula I on account of their favourable properties are those wherein $R_1$ is hydrogen, X (preferably in the 6-position)is hydrogen, methyl, halogen (especially chlorine) or methoxy, $R^3$ (preferably in the 3-position) is phenyl, or halo- or alkoxy-substituted phenyl (especially 4-fluorophenyl), and $R^2$ is hydrogen. Specific preferred compounds include

6-methoxy-3-phenyl-1,2,3,4-tetrahydrocarbazole,
6-chloro-3-(4-fluorophenyl)-1,2,3,4-tetrahydrocarbazole,
6-methyl-3-phenyl-1,2,3,4-tetrahydrocarbazole, and
6-chloro-3-(4-methoxyphenyl)-1,2,3,4-tetrahydrocarbazole.

In the following discussions of specific tests, the test compound of formula I (hereinafter simply referred to as "test compound") is 6-methoxy-3-phenyl-1,2,3,4-tetrahydrocarbazole.

In the rat muricide test (based on the method of Horovitz et al., Int. J. Neuropharmacol., 5: 405-411 (1966)), the following comparative $ED_{50}$ values were obtained on intraperitoneal administration:

| Compound | $ED_{50}$ (mg./kg.) |
|---|---|
| Test compound | 1.7* |
| Imipramine | 6.4 |
| Amitriptyline | 5.2 |

*This dosage level is significantly lower than those that cause changes in behaviour, in neurologic function or in autonomic function, in particular decreased spontaneous motor activity and decreased tail elevation. Such changes occur at dosage levels of about 100-1000 mg./kg (i.p.), about $50xED_{50}$ to $500xED_{50}$. In contrast, imipramine caused mydriasis and decreases in spontaneous motor activity, in muscle tone and in tail elevation at 30 mg./kg (i.p.), only about $5xED_{50}$. Thus, in this test the test compound shows a high therapeutic ratio.

The test compound and imipramine, both of which are active in ameliorating abnormal behaviour of beagles at 10 mg./kg. po, were evaluated also for effects on behaviour, neurologic and autonomic function in beagles with normal behaviour.

The test compound at 30 and 60 mg./kg. po and imipramine

at 10 and 30 mg./kg. po were administered to separate groups each of four dogs. All animals were then evaluated for up to 6 hours for changes in behaviour, neurologic or autonomic function according to a modification of the method of Irwin ["Drug screening and evaluation of new drugs in animals", pp. 36-56 in "Animal and Clinical Pharmacologic Techniques in Drug Evaluation", Editors: J.M. Nodine and P.E. Seigler, Chicago, Yearbook Medical Publisher Inc., 1964.]

The test compound did not affect behaviour, neurologic or autonomic function of beagles at doses up to 60 mg./kg. po; this dose is 6 times the MED (10 mg./kg. po) for improvement of abnormal behaviour in beagles. In contrast, imipramine caused tremors and mydriasis at its MED in abnormal dogs (10 mg./kg. po), and decreased motor activity and passivity at only three times its MED (30 mg./kg. po). Thus, the margin between doses producing improvement and those causing side effects in this species is greater with the test compound than with imipramine.

CLAIMS

1. A phenyl-1,2,3,4-tetrahydrocarbazole of the formula

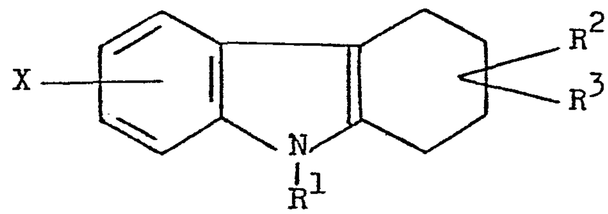

wherein X is a hydrogen or halogen atom or a nitro, $NR^4R^5$, lower alkyl, hydroxy, lower alkoxy or trifluoromethyl group;

$R^1$ is a hydrogen atom or a lower alkyl, $NR^4R^5$-substituted lower alkyl or $NR^4R^5$-CO group;

$R^3$ is a phenyl or X-substituted phenyl group, where X is as defined above;

$R^2$ is a hydrogen atom or an $NR^4R^5$-substituted alkyl, cyano, $NR^4R^5$-CO or COOR group, where R is a hydrogen atom or a lower alkyl group;

with the proviso that $R^2$ and $R^3$ are located on the same carbon atom;

and $R^4$ and $R^5$, which may be the same or different, are hydrogen atoms or lower alkyl groups;

where the term "lower" indicates groups having 1 to 6 carbon atoms;

and the pharmaceutically acceptable salts of those compounds having an acidic or basic group.

2. A compound as claimed in claim 1 wherein X is a methoxy group or a hydrogen or halogen atom, $R^1$ is a hydrogen atom and $R^3$ is a phenyl or 4-fluorophenyl group.

3. 6-Methoxy-3-phenyl-1,2,3,4-tetrahydrocarbazole,

6-Chloro-3-(4-fluorophenyl)-1,2,3,4-tetrahydrocarbazole,

6-Methyl-3-phenyl-1,2,3,4-tetrahxdrocarbazole, and

6-Chloro-3-(4-methoxyphenyl)-1,2,3,4-tetrahydrocarbazole.

4. A process for the preparation of a compound claimed in claim 1 or a pharmaceutically acceptable salt of a compound having an acidic or a basic group, which comprises either

(a) condensing a phenylhydrazine of the formula

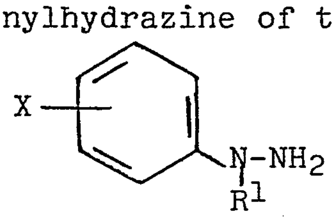

II

with a cyclohexanone of the formula

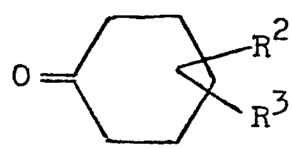

III

in an acidic medium at moderately elevated temperature,

or

(b) cyclising the intermediary phenylhydrazone of the formula

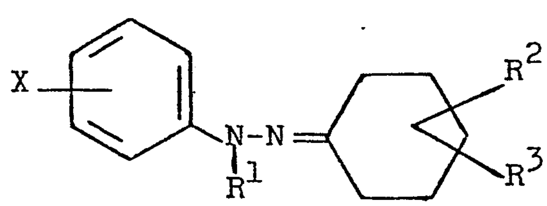

IV

in an acidic medium at elevated temperature;

wherein, in formulae II, III and IV, X, $R^1$, $R^2$ and $R^3$ are as defined in claim 1;

and, for the preparation of a pharmaceutically acceptable salt of a compound of the formula I having an acidic or basic group, isolating said compound as said salt.

5. A process as claimed in claim 14 wherein the reactants of the formula II and III or the phenylhydrazone of the formula IV are heated in an acidic medium, preferably in ethanolic hydrochloric acid or in acetic acid.

6. A process as claimed in claim 4 or claim 5 wherein the phenylhydrazine of the formula II or the phenylhydrazone of the formula IV has one of the following features:

(i) Substituent X is a nitro group and this after process (a) or (b) is reduced to an unsubstituted amino group;

(ii) Substituent X is a lower alkoxy group, and this is dealkylated after the process (a) or (b) to a hydroxy group;

(iii) Substituent $R^1$ is a hydrogen atom and, after the process (a) or (b), the product of the formula I wherein $R^1$ is a hydrogen atom is N-alkylated to introduce a lower alkyl or $NR^4R^5$-substituted lower alkyl group, wherein $R^4$ and $R^5$ are as defined in claim 1;

(iv) Substituent $R^1$ is a hydrogen atom and, after the process (a) or (b), the product of the formula I wherein $R^1$ is a hydrogen atom is reacted with chlorosulfonyl isocyanate or with a lower alkyl isocyanate, followed by

alkaline hydrolysis of the 9-chlorosulfonylcarbamoyl compound when chlorosulfonylisocyanate has been used, to yield a compound of the formula I wherein the group $R^1$ is a $CONH_2$ or $CONHR^4$ group, wherein $R^4$ is a lower alkyl group;

(v) Substituent $R^2$ in the cyclohexanone of the formula III is a cyano group and, after the process (a) or (b), the resulting compound of the formula I wherein $R^2$ is a cyano group is subjected to a process of hydration or hydrolysis wherein the group $R^2$ is hydrated to a group $CONH_2$ or hydrolysed to a group COOH;

(vi) Substituent $R^2$ in the cyclohexanone of the formula III is a cyano group and, after the process (a) or (b), the resulting compound of the formula I wherein $R^2$ is a cyano group is subjected to hydrolysis and esterification of the resulting carboxylic acid with a lower alkanol;

(vii) Substituent $R^2$ in the cyclohexanone of the formula III is a cyano group and, after the process (a) or (b), the resulting compound of the formula I wherein $R^2$ is a cyano group is subjected to reduction to yield a product of the formula I wherein $R^2$ is a group $CH_2NH_2$.

7. Pharmaceutical compositions containing, as active ingredient, at least one compound of the formula I defined in claim 1, or a pharmaceutically acceptable salt of such a compound having an acidic or basic group, together with a pharmaceutical carrier or excipient.

8. Compositions as claimed in claim 7 in the form of dosage units, preferably containing from 6 to 100 mg. of active ingredient.

9. Compositions as claimed in claim 7 or 8 wherein the active ingredient is as claimed in claim 2 or claim 3.

10. A process for the preparation of a pharmaceutical composition as claimed in any of claims 7 to 9 which comprises bringing, as active ingredient, at least one compound of the formula I defined in claim 1 or a pharmaceutically acceptable salt of such a compound having an acidic or basic group, into a form suitable for therapeutic administration, preferably by admixing with a pharmaceutical carrier or excipient.

0073849

European Patent Office

# EUROPEAN SEARCH REPORT

Application number

EP 81106891.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US - A - 4 254 134 (FLIEDNER) (03-03-1981) * Column 2, lines 8-45; column 7; column 8, lines 1-19; column 11, lines 40-42 * -- | 1,4,7 | C 07 D 209/86<br>C 07 D 209/88<br>A 61 K 31/40 |
| A | US - A - 4 172 834 (MOORADIAN) * Column 11, lines 45-64; column 5, lines 6-12 * -- | 1,4 | |
| A | US - A - 3 931 222 (CROSS) * Column 1, lines 15-51; column 3, line 68 - column 4, line 19 * ---- | 1,4,7 | |

TECHNICAL FIELDS SEARCHED (Int.Cl. 3)

C 07 D 209/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| VIENNA | 28-04-1982 | ONDER |

EPO Form 1503.1 06.78